# EUROPEAN PATENT APPLICATION

(11) **EP 2 543 678 A1**
(43) Date of publication of application: **09.01.2013**
(21) Application number: 11305888.7
(22) Date of filing: 08.07.2011
(51) Int. Cl.: C07K 16/36, A61K 39/395, A61P 7/02

(54) **Antibodies for the treatment and prevention of thrombosis**

(71) Applicant: INSERM (Institut National de la Santé et de la Recherche Médicale), 75013 Paris (FR)
(72) Inventor: The designation of the inventor has not yet been filed
(74) Representative: Hirsch, Denise Marie

(57) **Abstract**

The present disclosure relates to an isolated monoclonal antibody that specifically binds to the C1 domain of human von Willebrand factor and competes for binding to the C1 domain of human von Willebrand factor with the antibody obtainable from hybridoma deposited as CNCM-I-4483. Particularly, said embodiment is selected from the group consisting of a murine antibody, a chimeric antibody, a humanized antibody, and a human antibody. Antibodies of the present disclosure are presented to be useful in an antithrombotic treatment to prevent the formation of thrombus, which can be either a non- occlusive thrombus or an occlusive thrombus.

## Description

### FIELD OF THE INVENTION:

The present invention relates to methods for the treatment and prevention of thrombosis.

### BACKGROUND OF THE INVENTION:

Acute coronary and cerebrovascular accidents are currently the first death cause in the world. In addition, the global incidence of recurrence and death in the 6 month post-treatment period after an acute coronary syndrome is still 8-15%. In the case of acute coronary syndrome with ST segment elevation, mechanical treatment with coronary angioplasty and introduction of a stent is highly efficient to urgently restore coronary artery flow, but does not prevent morbidity/mortality for about 15% of patients in the next 6 months.

The central importance of platelets in the development of venous and arterial thrombosis is well established. Indeed excessive accumulation of platelets at sites of atherosclerotic plaque rupture is the crucial pathogenic event that is responsible for the development of the acute coronary syndrome, stroke and the ischemic complications of peripheral vascular disease.

Until recently, aspirin was the only antiplatelet agent in widespread clinical use for the prevention and treatment of CVD. Today it still remains the gold standard in terms of antiplatelet therapy both for acute ischemic syndromes as well as for secondary prevention of cardiovascular events. The second most common antiplatelet drug is clopidogrel (Plavix®), a P2Y12 ADP receptor antagonist. Clopidogrel has been used on a large scale as an antiplatelet drug since the mid-1990s and compared to aspirin, it reduces the occurrence of serious vascular event by an additional 9%. However, the antithrombotic effect of clopidogrel is still weak, only slightly better than aspirin mostly in high-risk patients. The third major group of antiplatelet drugs are inhibitors of platelet receptor GPIIbIIIa, commonly known as the final common pathway of platelet aggregation. Indeed, regardless of the primary thrombogenic stimulus, all pathways converge to the activation of GPIIbIIIa which plays a major role in promoting thrombus growth through its binding to fibrinogen and/or von Willebrand factor. The fist GPIIbIIIa inhibitor developed was abciximab (ReoPro®), the Fab fragment of a humanized murine monoclonal antibody. In addition to this antibody, other anti GPIIbIIIa inhibitors include epitifibatide (Integrillin®), a cyclic peptide and tirofiban (Aggrestat®), a non-peptide tryrosine derivative. Intravenous GPIIbIIIa inhibitors proved to be powerful antiplatelet agents and provided significant clinical benefit in patients undergoing coronary interventions such as percutaneous coronary intervention although increased bleeding risk was also associated with these inhibitors. But the major problems arose with the development of oral GPIIbIIIa inhibitors who proved to be unsafe, leading to increased mortality due to major bleeding.

The limitations of current anti-platelet drugs underscore the need for new antiplatelet agents.

Von Willebrand factor (VWF) is considered a good target to inhibit thrombosis. In normal hemostasis and thrombosis, platelets adhere to the subendothelium of damaged blood vessels through an interaction with von Willebrand factor (VWF), which forms a bridge between collagen within the damaged vessel wall and the platelet receptor glycoprotein Ib (GPIb). The VWF-GPIb interaction is transient and results in rolling and slowing down of platelets. During this rolling phase, inside-out signaling leads to activation of GPIIbIIIa and subsequent platelet aggregation through binding of GPIIbIIIa to fibrinogen and/or VWF. However agents effective to inhibit the the interaction of VWF with GPIIbIIIa has not yet been inbestigated for the treatment and prevention of thrombosis.

### SUMMARY OF THE INVENTION:

The present invention relates to an isolated monoclonal antibody that specifically binds to the C1 domain of human von Willebrand factor and competes for binding to the C1 domain of human von Willebrand factor with the antibody obtainable from hybridoma deposited as CNCM-I-4483. In a particular embodiment, said antibody is selected from the group consisting of a murine antibody, a chimeric antibody, a humanized antibody, and a human antibody. Antibodies of the present invention are presented to be useful in an antithrombotic treatment to prevent the formation of thrombus, which can be either a non-occlusive thrombus or an occlusive thrombus.

### DETAILED DESCRIPTION OF THE INVENTION:

Von Willebrand factor (VWF) is considered a good target to inhibit thrombosis. During thrombus formation, VWF interacts with collagen and platelet glycoprotein IIbIIIa (GPIIbIIIa) via its A3 domain and its C1 domain, respectively. The inventors have produced an anti human-VWF monoclonal antibody that can completely inhibit binding of VWF to GPIIbIIIa (mAb9) in vitro (Fressinaud E et al, The Journal of laboratory and clinical medicine, Vol 112, pages 58-67). In order to study the effect of this antibody in the formation of thrombi in vivo, they have developed a mouse model to test anti-thrombotic drugs targeting human VWF. This model is based on the expression of human VWF with a complete murine A1 domain insertion (hVWFmA1) in VWF deficient mice by hydrodynamic injection. Functionality of the hVWFmA1 chimera was assessed by the formation of occlusive thrombi in mesenteric vessels after 7.5% ferric chloride treatment. No significant difference was observed in the time to occlusion in vessels of animals expressing hVWFmA1 or wild type murine VWF after hydrodynamic injection. The antibody recognized the hVWFmA1 chimera as well as human VWF. The inventors next evaluated the effect of the antibody by injecting 100 µg of mAb, minutes before induction of thrombosis. Venous occlusion was observed in 75% of animals pretreated with mAb9. Arterial occlusion occurred only in 37.5% of animals pretreated with mAb9. When pretreated with a non-relevant antibody as a control, 100% of vessels (veins and arterioles) occluded. The results suggest a potential anti-thrombotic role of antibodies inhibiting binding of VWF to GPIIbIIIa such as mAb9, and more generally to agent inhibiting binding of VWF to GPIIbIIIa.

### Definitions:

Throughout the specification, several terms are employed and are defined in the following paragraphs.

The term "vWF" has its general meaning in the art and refers to the human von Willebrand factor (vWF). vWF is composed of several homologous domains each covering different functions: its "C1 domain" interacts with GPIIbIIIa (Girma JP, Kalafatis M, Piétu G, Lavergne JM, Chopek MW, Edgington TS, Meyer D.Mapping of distinct von Willebrand factor domains interacting with platelet GPIb and GPIIb/IIIa and with collagen using monoclonal antibodies. Blood. 1986 May;67(5):1356-66.; Veyradier A, Jumilly AL, Ribba AS, Obert B, Houllier A, Meyer D, Girma JP. New assay for measuring binding of platelet glycoprotein IIb/IIIa to unpurified von Willebrand factor. Thromb Haemost. 1999 Ju1;82(1):134-9.; Denis C, Williams JA, Lu X, Meyer D, Baruch D. Solid-phase von Willebrand factor contains a conformationally active RGD motif that mediates endothelial cell adhesion through the alpha v beta 3 receptor. Blood. 1993 Dec 15;82(12):3622-30.).

According to the present invention, "antibody" or "immunoglobulin" have the same meaning, and will be used equally in the present invention. The term "antibody" as used herein refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies and antibody fragments. In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. An antigen-binding site, therefore, includes six CDRs, comprising the CDR set from each of a heavy and a light chain V region. Framework Regions (FRs) refer to amino acid sequences interposed between CDRs.

The term "chimeric antibody" refers to an antibody which comprises a VH domain and a VL domain of an antibody, and a CH domain and a CL domain of a human antibody.

According to the invention, the term "humanized antibody" refers to an antibody having variable region framework and constant regions from a human antibody but retains the CDRs of a previous non human antibody.

The term "Fab" denotes an antibody fragment having a molecular weight of about 50,000 and antigen binding activity, in which about a half of the N-terminal side of H chain and the entire L chain, among fragments obtained by treating IgG with a protease, papaine, are bound together through a disulfide bond.

The term "F(ab')2" refers to an antibody fragment having a molecular weight of about 100,000 and antigen binding activity, which is slightly larger than the Fab bound via a disulfide bond of the hinge region, among fragments obtained by treating IgG with a protease, pepsin.

The term "Fab' " refers to an antibody fragment having a molecular weight of about 50,000 and antigen binding activity, which is obtained by cutting a disulfide bond of the hinge region of the F(ab')2.

A single chain Fv ("scFv") polypeptide is a covalently linked VH::VL heterodimer which is usually expressed from a gene fusion including VH and VL encoding genes linked by a peptide-encoding linker. "dsFv" is a VH::VL heterodimer stabilised by a disulfide bond. Divalent and multivalent antibody fragments can form either spontaneously by association of monovalent scFvs, or can be generated by coupling monovalent scFvs by a peptide linker, such as divalent sc(Fv)2.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

By "purified" and "isolated" it is meant, when referring to an antibody according to the invention or to a nucleotide sequence, that the indicated molecule is present in the substantial absence of other biological macromolecules of the same type. The term "purified" as used herein preferably means at least 75% by weight, more preferably at least 85% by weight, more preferably still at least 95% by weight, and most preferably at least 98% by weight, of biological macromolecules of the same type are present. An "isolated" nucleic acid molecule which encodes a particular polypeptide refers to a nucleic acid molecule which is substantially free of other nucleic acid molecules that do not encode the polypeptide; however, the molecule may include some additional bases or moieties which do not deleteriously affect the basic characteristics of the composition.

### Antibodies of the invention:

The present invention gives a publicly available source of the specific monoclonal antibody mAb9. Indeed, a mAb9 producing hybridoma has been deposited at the Collection Nationale de Cultures de Microorganismes (CNCM, Institut Pasteur, 25 rue du Docteur Roux, 75724 Paris Cedex 15, France), in accordance with the terms of Budapest Treaty, on the May 9^{th}, 2011. The deposited hybridoma has CNCM deposit number I-4483.

Accordingly, an aspect of the present invention provides an isolated monoclonal antibody that specifically binds to the C1 domain of human von Willebrand factor and competes for binding to C1 domain of human von Willebrand factor with the antibody obtainable from hybridoma deposited as CNCM-I-4483.

In a particular embodiment, said antibody is selected from the group consisting of a murine antibody, a chimeric antibody, a humanized antibody, and a human antibody

In another embodiment the antibody of the invention comprises a variable light chain (VL) comprising the CDRs of the VL chain of the antibody obtainable from hybridoma deposited as CNCM-I-4483.

In another embodiment the antibody of the invention comprises a variable heavy chain (VH) comprising the CDRs of the VH chain of the antibody obtainable from hybridoma deposited as CNCM-I-4483.

In another embodiment the antibody of the invention comprises a variable light chain (VL) comprising the CDRs of the VL chain of the antibody obtainable from hybridoma deposited as CNCM-I-4483 and a variable heavy chain (VH) comprising the CDRs of the VH chain of the antibody obtainable from hybridoma deposited as CNCM-I-4483.

In another embodiment the antibody of the invention comprises the VL chain of the antibody obtainable from hybridoma deposited as CNCM-I-4483 and the VH chain of the antibody obtainable from hybridoma deposited as CNCM-I-4483.

In another embodiment, the antibody of the invention is a chimeric antibody, which comprises the variable domains of the antibody obtainable from hybridoma deposited as CNCM-I-4483.

In another embodiment, the antibody of the invention is a humanized antibody. In particular, in said humanized antibody, the variable domain comprises human acceptor frameworks regions, and optionally human constant domain where present, and the CDRs of the antibody obtainable from hybridoma deposited as CNCM-I-4483.

A further aspect of the invention thus relates to a murine monoclonal antibody (mAb9) obtainable from the hybridoma available under CNCM deposit number I-4483.

The invention further provides fragments said antibodies which include but are not limited to Fv, Fab, F(ab')2, Fab', dsFv, scFv, sc(Fv)2 and diabodies.

### Competitive binding assays:

The present invention thus relates to pan isolated monoclonal antibody that specifically binds to C1 domain of human von Willebrand factor and competes for binding to C1 domain of human von Willebrand factor with the antibody obtainable from hybridoma deposited as CNCM-I-4483.

Epitope binning can be used to identify antibodies that fall within the scope of the claimed invention. Epitope binning refers to the use of competitive binding assays to identity pairs of antibodies that are, or are not, capable of binding HER3 simultaneously, thereby identifying pairs of antibodies that bind to the same or overlapping epitopes on HER3. Epitope binning experiments provide evidence that antigenically distinct epitopes are present. Competition for binding can be evaluated for any pair of antibodies or fragments. For example, using the appropriate detection reagents, the binding specificity of antibodies or binding fragments from any source can be compared to the binding specificity of the monoclonal antibodies disclosed herein. Epitope binning can be performed with "isolated antibodies" or with cell culture supernatants. Frequently, binning is performed with first round clonal supernatants to guide the choice of clones to be developed further. The antibodies to be compared should be substantially homogeneous antigen binding domains. In the case of "bispecific" or "bifunctional" antibodies the binding specificity of the two different binding sites need to be evaluated or binned independently.

The antibodies of the present invention may be assayed for specific binding by any method known in the art. Many different competitive binding assay format(s) can be used for epitope binning. The immunoassays which can be used include, but are not limited to, competitive assay systems using techniques such western blots, radioimmunoassays, ELISA, "sandwich" immunoassays, immunoprecipitation assays, precipitin assays, gel diffusion precipitin assays, immunoradiometric assays, fluorescent immunoassays, protein A immunoassays, and complement-fixation assays. Such assays are routine and well known in the art (see, e.g., Ausubel et al., eds, 1994 Current Protocols in Molecular Biology, Vol. 1, John Wiley & sons, Inc., New York). For example, the BIACOREⓇ (GE Healthcare, Piscaataway, NJ) is one of a variety of surface plasmon resonance assay formats that are routinely used to epitope bin panels of monoclonal antibodies. Additionally, routine cross-blocking assays such as those described in Antibodies, A Laboratory Manual, Cold Spring Harbor Laboratory, Ed Harlow and David Lane, 1988, can be performed.

### Methods of producing antibodies of the invention:

The antibodies of the invention may be produced by any technique known in the art, such as, without limitation, any chemical, biological, genetic or enzymatic technique, either alone or in combination.

Knowing the amino acid sequence of the desired sequence, one skilled in the art can readily produce said antibodies, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions. Alternatively, antibodies of the invention can be synthesized by recombinant DNA techniques well-known in the art. For example, antibodies can be obtained as DNA expression products after incorporation of DNA sequences encoding the antibodies into expression vectors and introduction of such vectors into suitable eukaryotic or prokaryotic hosts that will express the desired antibodies, from which they can be later isolated using well-known techniques.

Accordingly, a further object of the invention relates to a nucleic acid sequence encoding an antibody according to the invention.

In a particular embodiment, the invention relates to a nucleic acid sequence encoding the VH domain of the antibody of the invention (e.g. the antibody obtainable from hybridoma deposited as CNCM-I-4483 (mAb9)) or the VL domain of the antibody of the invention (e.g. the antibody obtainable from hybridoma deposited as CNCM-I-4483 (mAb9)).

Typically, said nucleic acid is a DNA or RNA molecule, which may be included in any suitable vector, such as a plasmid, cosmid, episome, artificial chromosome, phage or a viral vector.

The terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence.

So, a further object of the invention relates to a vector comprising a nucleic acid of the invention.

Such vectors may comprise regulatory elements, such as a promoter, enhancer, terminator and the like, to cause or direct expression of said antibody upon administration to a subject. Examples of promoters and enhancers used in the expression vector for animal cell include early promoter and enhancer of SV40 (Mizukami T. et al. 1987), LTR promoter and enhancer of Moloney mouse leukemia virus (Kuwana Y et al. 1987), promoter (Mason JO et al. 1985) and enhancer (Gillies SD et al. 1983) of immunoglobulin H chain and the like.

Any expression vector for animal cell can be used, so long as a gene encoding the human antibody C region can be inserted and expressed. Examples of suitable vectors include pAGE107 (Miyaji H et al. 1990), pAGE103 (Mizukami T et al. 1987), pHSG274 (Brady G et al. 1984), pKCR (O'Hare K et al. 1981), pSG1 beta d2-4-(Miyaji H et al. 1990) and the like.

Other examples of plasmids include replicating plasmids comprising an origin of replication, or integrative plasmids, such as for instance pUC, pcDNA, pBR, and the like.

Other examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

A further object of the present invention relates to a host cell which has been transfected, infected or transformed by a nucleic acid and/or a vector according to the invention.

The term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. A host cell that receives and expresses introduced DNA or RNA bas been "transformed".

The nucleic acids of the invention may be used to produce an antibody of the invention in a suitable expression system. The term "expression system" means a host cell and compatible vector under suitable conditions, e.g. for the expression of a protein coded for by foreign DNA carried by the vector and introduced to the host cell.

Common expression systems include E. coli host cells and plasmid vectors, insect host cells and Baculovirus vectors, and mammalian host cells and vectors. Other examples of host cells include, without limitation, prokaryotic cells (such as bacteria) and eukaryotic cells (such as yeast cells, mammalian cells, insect cells, plant cells, etc.). Specific examples include E.coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from lymphoblasts, fibroblasts, embryonic cells, epithelial cells, nervous cells, adipocytes, etc.). Examples also include mouse SP2/0-Agl4 cell (ATCC CRL1581), mouse P3X63-Ag8.653 cell (ATCC CRL1580), CHO cell in which a dihydrofolate reductase gene (hereinafter referred to as "DHFR gene") is defective (Urlaub G et al; 1980), rat YB2/3HL.P2.G11.16Ag.20 cell (ATCC CRL1662, hereinafter referred to as "YB2/0 cell"), and the like.

The present invention also relates to a method of producing a recombinant host cell expressing an antibody according to the invention, said method comprising the steps of: (i) introducing in vitro or ex vivo a recombinant nucleic acid or a vector as described above into a competent host cell, (ii) culturing in vitro or ex vivo the recombinant host cell obtained and (iii), optionally, selecting the cells which express and/or secrete said antibody. Such recombinant host cells can be used for the production of antibodies of the invention.

In another particular embodiment, the method comprises the steps of:
(i) culturing the hybridoma deposited as CNCM-I-4483 under conditions suitable to allow expression of mAb9 antibody; and
(ii) recovering the expressed antibody.

Antibodies of the invention are suitably separated from the culture medium by conventional immunoglobulin purification procedures such as, for example, protein A-Sepharose, hydroxylapatite chromatography, gel electrophoresis, dialysis, or affinity chromatography.

In a particular embodiment, the human chimeric antibody of the present invention can be produced by obtaining nucleic sequences encoding VL and VH domains as previously described, constructing a human chimeric antibody expression vector by inserting them into an expression vector for animal cell having genes encoding human antibody CH and human antibody CL, and expressing the coding sequence by introducing the expression vector into an animal cell.

As the CH domain of a human chimeric antibody, it may be any region which belongs to human immunoglobulin, but those of IgG class are suitable and any one of subclasses belonging to IgG class, such as IgG1, IgG2, IgG3 and IgG4, can also be used. Also, as the CL of a human chimeric antibody, it may be any region which belongs to Ig, and those of kappa class or lambda class can be used.

Methods for producing chimeric antibodies involve conventional recombinant DNA and gene transfection techniques are well known in the art (See Morrison SL. et al. (1984) and patent documents US5,202,238; and US5,204, 244).

The humanized antibody of the present invention may be produced by obtaining nucleic acid sequences encoding CDR domains, as previously described, constructing a humanized antibody expression vector by inserting them into an expression vector for animal cell having genes encoding (i) a heavy chain constant region identical to that of a human antibody and (ii) a light chain constant region identical to that of a human antibody, and expressing the genes by introducing the expression vector into an animal cell.

The humanized antibody expression vector may be either of a type in which a gene encoding an antibody heavy chain and a gene encoding an antibody light chain exists on separate vectors or of a type in which both genes exist on the same vector (tandem type). In respect of easiness of construction of a humanized antibody expression vector, easiness of introduction into animal cells, and balance between the expression levels of antibody H and L chains in animal cells, humanized antibody expression vector of the tandem type is preferred (Shitara K et al. 1994). Examples of tandem type humanized antibody expression vector include pKANTEX93 (WO 97/10354), pEE18 and the like.

Methods for producing humanized antibodies based on conventional recombinant DNA and gene transfection techniques are well known in the art (See, e. g., Riechmann L. et al. 1988; Neuberger MS. et al. 1985). Antibodies can be humanized using a variety of techniques known in the art including, for example, CDR-grafting (EP 239,400; PCT publication WO91/09967; U.S. Pat. Nos. 5,225,539; 5,530,101; and 5,585,089), veneering or resurfacing (EP 592,106; EP 519,596; Padlan EA (1991); Studnicka GM et al. (1994); Roguska MA. et al. (1994)), and chain shuffling (U.S. Pat. No.5,565,332). The general recombinant DNA technology for preparation of such antibodies is also known (see European Patent Application EP 125023 and International Patent Application WO 96/02576).

The Fab of the present invention can be obtained by treating an antibody which specifically reacts with human HER3 with a protease, papaine. Also, the Fab can be produced by inserting DNA encoding Fab of the antibody into a vector for prokaryotic expression system, or for eukaryotic expression system, and introducing the vector into a procaryote or eucaryote (as appropriate) to express the Fab.

The F(ab')2 of the present invention can be obtained treating an antibody which specifically reacts with human HER3 with a protease, pepsin. Also, the F(ab')2 can be produced by binding Fab' described below via a thioether bond or a disulfide bond.

The Fab' of the present invention can be obtained treating F(ab')2 which specifically reacts with human HER3 with a reducing agent, dithiothreitol. Also, the Fab' can be produced by inserting DNA encoding Fab' fragment of the antibody into an expression vector for prokaryote, or an expression vector for eukaryote, and introducing the vector into a prokaryote or eukaryote (as appropriate) to perform its expression.

The scFv of the present invention can be produced by obtaining cDNA encoding the VH and VL domains as previously described, constructing DNA encoding scFv, inserting the DNA into an expression vector for prokaryote, or an expression vector for eukaryote, and then introducing the expression vector into a prokaryote or eukaryote (as appropriate) to express the scFv. To generate a humanized scFv fragment, a well known technology called CDR grafting may be used, which involves selecting the complementary determining regions (CDRs) from a donor scFv fragment, and grafting them onto a human scFv fragment framework of known three dimensional structure (see, e. g., WO98/45322; WO 87/02671; US5,859,205; US5,585,089; US4,816,567; EP0173494).

Amino acid sequence modification(s) of the antibodies described herein are contemplated. For example, it may be desirable to improve the binding affinity and/or other biological properties of the antibody. It is known that when a humanized antibody is produced by simply grafting only CDRs in VH and VL of an antibody derived from a non-human animal in FRs of the VH and VL of a human antibody, the antigen binding activity is reduced in comparison with that of the original antibody derived from a non-human animal. It is considered that several amino acid residues of the VH and VL of the non-human antibody, not only in CDRs but also in FRs, are directly or indirectly associated with the antigen binding activity. Hence, substitution of these amino acid residues with different amino acid residues derived from FRs of the VH and VL of the human antibody would reduce of the binding activity. In order to resolve the problem, in antibodies grafted with human CDR, attempts have to be made to identify, among amino acid sequences of the FR of the VH and VL of human antibodies, an amino acid residue which is directly associated with binding to the antibody, or which interacts with an amino acid residue of CDR, or which maintains the three-dimensional structure of the antibody and which is directly associated with binding to the antigen. The reduced antigen binding activity could be increased by replacing the identified amino acids with amino acid residues of the original antibody derived from a non-human animal.

Modifications and changes may be made in the structure of the antibodies of the present invention, and in the DNA sequences encoding them, and still obtain a functional molecule that encodes an antibody with desirable characteristics.

In making the changes in the amino sequences, the hydropathic index of amino acids may be considered. The importance of the hydropathic amino acid index in conferring interactive biologic function on a protein is generally understood in the art. It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of their hydrophobicity and charge characteristics these are: isoleucine (+4.5); valine (+4.2); leucine (+3.8) ; phenylalanine (+2.8); cysteine/cystine (+2.5); methionine (+1.9); alanine (+1.8); glycine (-0.4); threonine (-0.7); serine (-0.8); tryptophane (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

A further object of the present invention also encompasses function-conservative variants of the antibodies of the present invention.

"Function-conservative variants" are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide which has at least 60 % amino acid identity as determined by BLAST or FASTA algorithms, preferably at least 75 %, more preferably at least 85%, still preferably at least 90 %, and even more preferably at least 95%, and which has the same or substantially similar properties or functions as the native or parent protein to which it is compared.

Two amino acid sequences are "substantially homologous" or "substantially similar" when greater than 80 %, preferably greater than 85 %, preferably greater than 90 % of the amino acids are identical, or greater than about 90 %, preferably grater than 95 %, are similar (functionally identical) over the whole length of the shorter sequence. Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.

For example, certain amino acids may be substituted by other amino acids in a protein structure without appreciable loss of activity. Since the interactive capacity and nature of a protein define the protein's biological functional activity, certain amino acid substitutions can be made in a protein sequence, and, of course, in its DNA encoding sequence, while nevertheless obtaining a protein with like properties. It is thus contemplated that various changes may be made in the antibodies sequences of the invention, or corresponding DNA sequences which encode said antibodies, without appreciable loss of their biological activity.

It is known in the art that certain amino acids may be substituted by other amino acids having a similar hydropathic index or score and still result in a protein with similar biological activity, i.e. still obtain a biological functionally equivalent protein.

As outlined above, amino acid substitutions are generally therefore based on the relative similarity of the amino acid side-chain substituents, for example, their hydrophobicity, hydrophilicity, charge, size, and the like. Exemplary substitutions which take various of the foregoing characteristics into consideration are well known to those of skill in the art and include: arginine and lysine; glutamate and aspartate; serine and threonine; glutamine and asparagine; and valine, leucine and isoleucine.

Another type of amino acid modification of the antibody of the invention may be useful for altering the original glycosylation pattern of the antibody.

By "altering" is meant deleting one or more carbohydrate moieties found in the antibody, and/or adding one or more glycosylation sites that are not present in the antibody.

Glycosylation of antibodies is typically N-linked. "N-linked" refers to the attachment of the carbohydrate moiety to the side chain of an asparagine residue. The tripeptide sequences asparagine-X-serine and asparagines-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. Addition of glycosylation sites to the antibody is conveniently accomplished by altering the amino acid sequence such that it contains one or more of the above-described tripeptide sequences (for N-linked glycosylation sites).

Another type of covalent modification involves chemically or enzymatically coupling glycosides to the antibody. These procedures are advantageous in that they do not require production of the antibody in a host cell that has glycosylation capabilities for N-or O-linked glycosylation. Depending on the coupling mode used, the sugar(s) may be attached to (a) arginine and histidine, (b) free carboxyl groups, (c) free sulfhydryl groups such as thoseof cysteine, (d) free hydroxyl groups such as those of serine, threonine, orhydroxyproline, (e) aromatic residues such as those of phenylalanine, tyrosine, or tryptophan, or (f) the amide group of glutamine. For example, such methods are described in WO87/05330.

Removal of any carbohydrate moieties present on the antibody may be accomplished chemically or enzymatically. Chemical deglycosylation requires exposure of the antibody to the compound trifluoromethanesulfonic acid, or an equivalent compound. This treatment results in the cleavage of most or all sugars except the linking sugar (N-acetylglucosamine or N-acetylgalactosamine), while leaving the antibody intact. Chemical deglycosylation is described by Sojahr H. et al. (1987) and by Edge, AS. et al. (1981). Enzymatic cleavage of carbohydrate moieties on antibodies can be achieved by the use of a variety of endo-and exo-glycosidases as described by Thotakura, NR. et al. (1987).

Another type of covalent modification of the antibody comprises linking the antibody to one of a variety of non proteinaceous polymers, eg. , polyethylene glycol, polypropylene glycol, or polyoxyalkylenes, in the manner set forth in US Patent Nos. 4,640, 835; 4,496, 689; 4,301, 144; 4,670, 417; 4,791, 192 or 4,179,337.

### Therapeutic methods and uses of the invention:

Antibodies of the present invention are presented to be useful in an antithrombotic treatment to prevent the formation of thrombus, which can be either a non-occlusive thrombus or an occlusive thrombus. Particularly, the antithrombotic treatment is envisaged to prevent arterial thrombus formation, such as acute coronary occlusion. The antibodies of the invention are further provided in a method of antithrombotic treatment to maintain the patency of diseased arteries, to prevent restenosis, such as after PCTA or stenting, to prevent thrombus formation in stenosed arteries, to prevent hyperplasia after angioplasty, atherectomy or arterial stenting, to prevent unstable angina, and generally to prevent or treat the occlusive syndrome in a vascular system.

Antibodies or fragments of the invention may be thus useful for the prevention of thrombosis, and particular venous and arterial thrombosis

Antibodies or fragments according to the invention may also be used to treat patients with acute coronary syndrome s, in particular by preventing further events in the coronary arteries.

Antibodies or fragments according to the invention may finally be used to prevent restenosis after vascular injury.

Antibodies of the invention may be also useful for thrombotic microangiopathy, thrombotic thrombocytopenic purpura or Schulman-Upshaw syndrome.

The term "thrombotic microangiopathy" describes syndromes of microangiopathic haemolytic anaemia, thrombocytopenia, and variable signs of organ impairment, due to platelet aggregation in the microcirculation.

The term "thrombotic thrombocytopenic purpura" (or Moschowitz disease) refers to a disorder of the blood-coagulation system, arising from deficiency or inhibition of the enzyme ADAMTS13, which is responsible for leaving large multimers of von Willebrand factor, and leading to haemolysis and end-organ damage.

The term "Schulman-Upshaw syndrome" refers to congenital thrombotic thrombocytopenic purpura.

Therapeutic antibodies according to the invention thus lead to the inhibition of the interaction between platelets and vWF, thereby inhibiting excessive adhesion and aggregation of platelets responsible for thrombosis.

Thus, an object of the invention relates to an antibody as defined above for the treatment of thrombosis, thrombotic microangiopathy, thrombotic thrombocytopenic purpura, Schulman-Upshaw syndrome. Such treatment may comprise the steps consisting in administering a subject in need thereof with a therapeutically effective amount of an antibody or fragment of the invention.

According to the invention, the term "subject" or "subject in need thereof" is intended for a human or non-human mammal (such as a rodent (mouse, rat), a feline, a canine, or a primate) affected or likely to be affected with von Willebrand factor dependent thrombosis, thrombotic microangiopathy, thrombotic thrombocytopenic purpura, Schulman-Upshaw syndrome, atherosclerosis or acute syndrome diseases. Preferably, the subject is a human.

The term "therapeutically effective amount" is meant for a sufficient amount of antibody in order to treat said disease, at a reasonable benefit/risk ratio applicable to any medical treatment. It will be understood, however, that the total daily usage of the antibodies and compositions of the present invention will be decided by the attending physician within the scope of sound medical judgment. The specific therapeutically effective dose level for any particular patient will depend upon a variety of factors including the disorder being treated and the severity of the disorder, activity of the specific antibody employed, the specific composition employed, the age, body weight, general health, sex and diet of the patient, the time of administration, route of administration, and rate of excretion of the specific antibody employed, the duration of the treatment, drugs used in combination or coincidental with the specific polypeptide employed, and like factors well known in the medical arts. For example, it is well known within the skill of the art to start doses of the compound at levels lower than those required to achieve the desired therapeutic effect and to gradually increase the dosage until the desired effect is achieved.

Antibodies of the invention may be used in combination with any other therapeutical strategy for treating thrombosis, thrombotic microangiopathy, thrombotic thrombocytopenic purpura, Schulman-Upshaw syndrome, atherosclerosis or acute syndrome diseases (e.g. plasmapharesis). The antibodies of the invention may be used alone or in combination with any suitable agent. For example, the antibodies of the invention may be combined with an anti-platelet agent such as aspirin, P2Y12 ADP receptor antagonist (e.g. clopidogrel) and GPIIbIIIa inhibitors. The antibodies of the invention may also be combined with thrombolytic agent or a recombinant variant or fragment thereof which is selected from the group consisting of staphylokinase, tissue plasminogen activator, streptokinase, single chain streptokinase, urokinase and acyl plasminogen-streptokinase complex.

In a further aspect of the invention the present invention relates to an agent effective to inhibit the binding of GPIIbIIIa to the C1 domain of human von Willebrand factor or use in the prevention or treatment of thrombosis.

Typically, the agent includes but is not limited to an antibody, a small organic molecule, an aptamer and a polypeptide.

In a particular embodiment the agent specifically binds to the C1 domain of human von Willebrand factor and competes for binding to C1 domain of human von Willebrand factor with the antibody obtainable from hybridoma deposited as CNCM-I-4483.

In a particular embodiment, the agent is an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas et al., 1996).

### Pharmaceutical compositions:

The nucleic acids, antibodies or fragments thereof and agents of the invention may be combined with pharmaceutically acceptable carriers, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The form of the pharmaceutical compositions, the route of administration, the dosage and the regimen naturally depend upon the condition to be treated, the severity of the illness, the age, weight, and sex of the patient, etc.

The pharmaceutical or therapeutic compositions of the invention can be formulated for a topical, oral, parenteral, intranasal, intravenous, intramuscular, subcutaneous or intraocular administration and the like.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

To prepare pharmaceutical compositions, an effective amount of the antibody may be dissolved or dispersed in a pharmaceutically acceptable carrier or aqueous medium.

The pharmaceutical forms suitable for injectable use include sterile aqueous solutions or dispersions, formulations including sesame oil, peanut oil or aqueous propylene glycol, and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of micro-organisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of micro-organisms.

An antibody of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of micro-organisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatine.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The antibodies of the invention may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds formulated for parenteral administration, such as intravenous or intramuscular injection, other pharmaceutically acceptable forms include, e.g. tablets or other solids for oral administration, time release capsules, and any other form currently used.

In certain embodiments, the use of liposomes and/or nanoparticles is contemplated for the introduction of antibodies into host cells. The formation and use of liposomes and/or nanoparticles are known to those of skill in the art.

Nanocapsules can generally entrap compounds in a stable and reproducible way. To avoid side effects due to intracellular polymeric overloading, such ultrafine particles (sized around 0.1 µm) are generally designed using polymers able to be degraded in vivo. Biodegradable polyalkyl-cyanoacrylate nanoparticles that meet these requirements are contemplated for use in the present invention, and such particles may be easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core. The physical characteristics of liposomes depend on pH, ionic strength and the presence of divalent cations.

The invention will be further illustrated by the following figures and examples. However, these examples and figures should not be interpreted in any way as limiting the scope of the present invention.

### FIGURES:

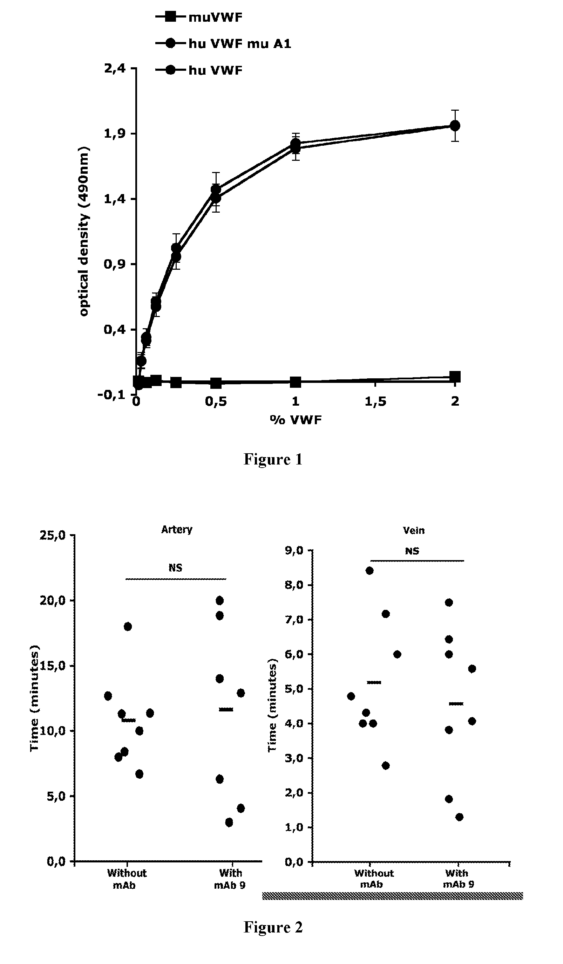

**Figure 1****: Interaction of mAb 9 with VWF variants.** Interaction of expressed huVWF (black circles), murine VWF (muVWF) (black boxes) and huVWFmuA1 (grey circles) with mAb 9 was measured in an ELISA sytem.
**Figure 2****: Role of mAb 9 in formation of a 30µm thrombus**. Mesenteric vessels of mice expressing huVWFmuA1 were exposed and treated with FeCl3 for 5 minutes. Mice were injected previously with rhodamine 6G to label the platelets and were either left untreated or injected with 100µg of mAb 9. Formation of thrombus was recorded and time necessary to build thrombus of 30µm size was recorded. Left panel: Time to first thrombus of 30µm in arterioles in untreated (black) and mAb 9 treated mice (grey). Right panel: Time to first thrombus of 30µm in veins in untreated (black) and mAb 9 treated mice (grey). Each dot represents one individual mouse. Statistics was done using Mann-Whitney-Wilcoxon rank test.

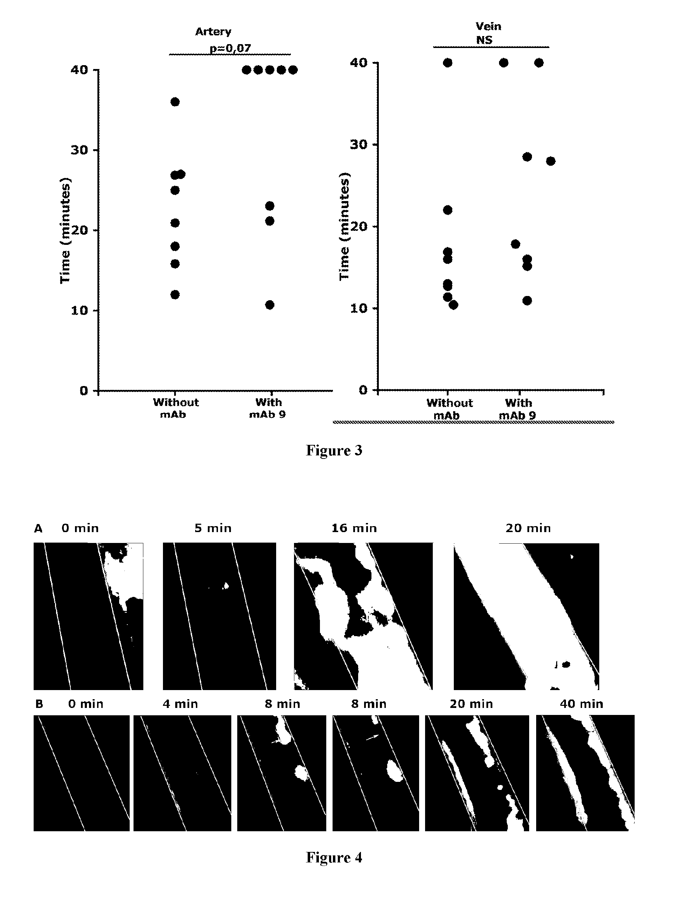

**Figure 3****: Role of mAb 9 in vessel occlusion.** Mesenteric vessels of mice expressing huVWFmuA1 were exposed and treated with FeCl3 for 5 minutes. Mice were injected previously with rhodamine 6G to label the platelets and were either left untreated or injected with 100µg of mAb 9. Formation of thrombus was recorded for 40 minutes after FeCl3 treatment and the time of occlusion of veins and arteries was recorded. Left panel: Occlusion time in arterioles of untreated (black) and mAb 9 treated mice (grey). Right panel: Occlusion time in veins of untreated (black) and mAb 9 treated mice (grey). Each dot represents one individual mouse. Statistics was done using Mann-Whitney-Wilcoxon rank test.
**Figure 4****: Effect of mAb 9 on thrombus formation in arterioles of mice.** Mesenteric vessels of mice expressing huVWFmuA1 were exposed and treated with FeCl3 for 5 minutes. Mice were injected previously with rhodamine 6G to label the platelets and were either left untreated or injected with 100µg of mAb 9. Formation of thrombus was recorded for 40 minutes after FeCl3. Panel A shows the sequence of thrombus formation in arteries of untreated mice and panel B shows the sequence of thrombus formation in arteries of mAb 9 treated mice. Vessel walls are represented by the white lines. Platelet accumulation can be visualized by the white/grey accumulation of material within the vessel. White arrows show the presence of the thrombus that was ripped off the vessel wall through embolization. Vessel occlusion is visible in the untreated mouse after 20 minutes while no vessel occlusion is reached in the mAb 9-treated mouse after 40 minutes.

### EXAMPLE: ROLE OF ANTI-HUMAN VON WILLEBRAND FACTOR MONOCLONAL ANTIBODY 9 IN THROMBUS FORMATION IN VIVO

### Introduction

Monoclonal antibody 9 (mAb 9) directed against human Von Willebrand Factor (huVWF) has been shown to inhibit the interaction of human VWF with platelet integrin glycoprotein α2β3 (Fressinaud E et al, The Journal of laboratory and clinical medicine, Vol 112, pages 58-67). Interaction of VWF with integrin α2β3 is crucial for the stabilization of the thrombi once formed. The purpose of this work was to explore the role of mAb 9 *in vivo* in the formation of the thrombus. However, huVWF is unable to interact with murine glycoprotein Ib (GPIb) expressed by murine platelets, and is therefore not functional in the mouse. We developed a model were human VWF was modified in order to interact with murine GPIb. Indeed the functional domain (the A1 domain) responsible for binding to platelet GPIb was replaced in huVWF by its murine counterpart (huVWFmuA1). Using hydrodynamic injection we were able to express transiently in VWF-deficient mice, a plasmid containing this chimeric VWF. Functionality of the molecule was validated using the bleeding test and the thrombosis in vivo model. Using this murine model we studied the role of mAb 9 in the formation of the thrombus *in vivo.*

### 1. Antibody mAb 9 recognizes the human VWF chimera

We first wanted to validate that the huVWFmuA1 molecule was recognized by mAb 9

### 1.1 Method

Mice deficient in VWF were injected with the PCDNA6 vectors (PCDNA6 huVWF, PCDNA6 huVWFmuA1 and PCDNA6 muVWF) via hydrodynamic injection. Plasmid DNA (150µg) was diluted in a saline solution (0.9% NaCl) and injected in the tail vein in a volume corresponding to 10% of the weight of the mouse (2ml for a 20g mouse) in five seconds. After twenty-four hours, mice were anesthetized and blood was collected in 0.138M trisodium citrate (1/10 volume). Plasma was prepared by centrifugation at 1500g for 20 minutes and aliquots were kept at -80°C until use. VWF antigen concentration in the plasma was measured by ELISA. Antigen level was expressed as percentage of VWF compared to a human plasma pool (considered as 100%). Interaction of mAb 9 with the plasma VWF variants was assessed by ELISA. 96 well-microplates were coated with 5µg/mL of mAb 9 at 4°C. Plates were blocked using PBS containing 3% bovine serum albumin (BSA) and 0.1% Tween-20 for 30 minutes at 37°C. Plasma samples were diluted on PBS 3% BSA and 0.1% Tween-20 to a initial concentration of 2% of VWF followed by serial dilutions. Plasma dilutions were incubated for 2 hours at 37°C. VWF bound was subsequently detected using mAb 418 coupled to horseradish peroxidase (recognition of mAb 418 of VWF is different from mAb 9 and does not include the domain A1 from VWF). Plate was revealed using o-PHENYLENEDIAMINE SIGMAⓇ fast tablet set. Reaction was stopped with H2SO4 IN and OD was read at a wavelength of 490 nanometers.

### 1.2 Results

We first checked that mAb 9 was able to recognize huVWFmuA1 expressed in the plasma of mice deficient on VWF after hydrodynamic injection. We used as positive control the plasma of mice expressing huVWF and as a negative control mice expressing murine VWF after hydrodynamic injection (Figure 1). Interaction of mAb 9 with huVWFmuA1 was similar as the one observed for huVWF. Binding was dose dependent and reached a plateau at concentrations similar to huVWF. No binding of mAb 9 was observed with murine VWF demonstrating the specificity of the interaction. This result shows that the domain recognized by mAb 9 remains unaltered in huVWFmuA1 therefore binding equally as to huVWF.

### 2. Role of mAb 9 in thrombus formation: Potential role in prevention of thrombosis

In order to evaluate the role of mAb 9 in thrombus formation *in vivo,* we used a FeCl₃ induced injury thrombosis model.

### 2.1 Method

Three to four weeks old mice (14g) deficient in VWF were injected hydrodynamically with the pLIVE plasmid containing huVWFmuA1 (150µg). Four days later, animals were anesthetized intraperitoneally with pentobarbital. They were then injected with 100µg of mAb 9 by retro-orbital injection or were left untreated. After 10 minutes, the mice were injected with 50µL of Rhodamine 6G dye to label platelets *in vivo* (10mg/mL) by retro-orbital injection. Immediately after, a midline abdominal incision was performed on each mouse and mesentery was delicately exposed. Under the reverse microscope a single arteriole was then selected based on size (100-130µm) and vessel exposure. A filter paper strip saturated with FeCl₃ (7.5%) was then applied for 5 minutes on the vessel in order to damage the endothelium. Thrombus formation was then recorded until vessel occlusion or for 40 minutes maximum after the application of the FeCl₃ using a Sony camera. Thrombus formation was analyzed using Archimede software (Microvision Instruments). The time required for initial thrombus formation (greater that 30µm) and occlusion of the vessel was recorded.

### 2.2 Results

### 2.2.1 Role of the mAb 9 in the time necessary to form a 30 µm thrombus

The average time necessary to form a 30µm thrombus in untreated mice was 5.2 ± 1.87 minutes after FeCl₃ in the veins and 10.8 ± 3.52 in arterioles (Figure 2). The time for the formation of 30µm thrombus was more homogeneous in the arteriole than in the vein. The formation of a 30µm was similar to controls delayed the arterioles and veins of mAb 9 pretreated mice, 11.6 ± 6.4 minutes and 4.6 ± 2.2 minutes respectively. Our results shows that treatment with mAb 9 does not alter initial thrombus formation in arterioles.

### 2.2.2 Role of the mAb 9 in vessel occlusion:

The final step of thrombus formation is the occlusion of the vessels after a gradual increase of the stabilized thrombi. 100% of untreated mice reached occlusion both in veins and arterioles within the 40 minutes observation period (Figure 3). Average occlusion time for controls was 22 ± 9.7 minutes in arteries and 17.8 ± 9.7 minutes in veins. We found that 6 out of 8 mAb 9 treated mice (75%) occluded in veins and only 3 out of 8 mAb 9-treated mice (37,5%) reached occlusion in arterioles (Figures 3 and 4). Altogether mAb 9 treated mice showed recurrent embolization of formed thrombi in arterioles (Figure 4, white arrows) No embolization process was seen in untreated mice. Interestingly, when occlusion occurred in arterioles of mAb 9 treated mice, it was the direct consequence of the embolization process with emboli provoking occlusion downstream of the injured part of the vessel. Our results show an important inhibitory effect in occlusive thrombus formation in arteries when mAb 9 is added preventively. Administration of non-relevant antibody or isotypic control did not alter occlusion time in mice expressing huVWFmuA1, showing that the effect observed is specific for mice treated with mAb 9.

### REFERENCES:

Throughout this application, various references describe the state of the art to which this invention pertains. The disclosures of these references are hereby incorporated by reference into the present disclosure.

## Claims

1. An isolated monoclonal antibody that specifically binds to the C1 domain of human von Willebrand factor and competes for binding to C1 domain of human von Willebrand factor with the antibody obtainable from hybridoma deposited as CNCM-I-4483.

2. The antibody according to claim 1 which is selected from the group consisting of a murine antibody, a chimeric antibody, a humanized antibody, and a human antibody.

3. The antibody according to claim 1 which comprises a variable heavy chain (VH) comprising the CDRs of the VH chain of the antibody obtainable from hybridoma deposited as CNCM-I-4483.

4. The antibody according to claim 3 which comprises a variable light chain (VL) comprising the CDRs of the VL chain of the antibody obtainable from hybridoma deposited as CNCM-I-4483.

5. The antibody according to claim 3 which comprises a variable light chain (VL) comprising the CDRs of the VL chain of the antibody obtainable from hybridoma deposited as CNCM-I-4483 and a variable heavy chain (VH) comprising the CDRs of the VH chain of the antibody obtainable from hybridoma deposited as CNCM-I-4483.

6. The antibody according to claim 3 which comprises the VL chain of the antibody obtainable from hybridoma deposited as CNCM-I-4483 and the VH chain of the antibody obtainable from hybridoma deposited as CNCM-I-4483.

7. The antibody according to claim 3 which is a chimeric antibody, which comprises the variable domains of the antibody obtainable from hybridoma deposited as CNCM-I-4483.

8. The antibody according to claim 3 which is a humanized antibody.

9. The antibody according to claim 3 which is the murine monoclonal antibody (mAb9) obtainable from the hybridoma available under CNCM deposit number I-4483.

10. A fragment of an antibody according to any of the preceding claims which is selected from the group consisting of Fv, Fab, F(ab')2, Fab', dsFv, scFv, sc(Fv)2 and diabodies.

11. A nucleic acid encoding the VH domain of the antibody of any claims 1 to 9 or the VL domain of the antibody of any claims 1 to 9.

12. A host cell which has been transfected, infected or transformed by a nucleic acid according to claim 11.

13. The antibody according to any of claims 1 to 10 or fragment according to claim 9 for use in a method for treating or preventing thrombosis.

14. A pharmaceutical composition comprising an antibody according to any of claims 1 to 9 or a fragment according to claim 10.
